# EUROPEAN PATENT APPLICATION

(11) **EP 4 134 124 A1**
(43) Date of publication of application: **15.02.2023**
(21) Application number: 21190721.7
(22) Date of filing: 11.08.2021
(51) Int. Cl.: A61N 1/05, A61B 17/34

(54) **IMPLANTABLE ELECTRODE COMPRISING AN EVACUATION CHANNEL**

(71) Applicant: BIOTRONIK SE & Co. KG, 12359 Berlin (DE)
(72) Inventor: Dörr, Thomas, 12437 Berlin (DE); Kolberg, Gernot, 12161 Berlin (DE); Jochum, Tobias, 13187 Berlin (DE)

(57) **Abstract**

The invention relates to an implantable electrode for a medical device, the implantable electrode (1) comprising an electrode shaft (7), an interior lumen (8) inside the electrode shaft (7) and an electrode pole (4, 5, 6) on an outer surface of the electrode shaft (7). According to an aspect of the invention, the electrode pole (4, 5, 6) comprises an opening (10) allowing a fluid communication between an outside of the electrode shaft (7) and the interior lumen (8), wherein the interior lumen (8) comprises a suction port (9) allowing to apply a negative pressure to the interior lumen (8).

## Description

The present invention relates to an implantable electrode for medical device according to the preamble of claim 1, to an electrode assembly comprising such an implantable electrode according to the preamble of claim 12, and to a medical device comprising such an implantable electrode according to the preamble of claim 13.

Upon implanting an electrode into a body of a patient, residual air often remains present between the electrode and the surrounding body tissue with which the electrode is intended to interact. These air inclusions result in an increased detection and/or stimulation threshold for a detection and/or stimulation electrode. The reason for these air inclusions is air being present in a delivery catheter used for implantation. This air is pushed by an electrode to be implanted by the delivery catheter in front of the electrode. If the electrode is to be implanted within the bloodstream of the patient, such air inclusions are less important since the blood pressure regularly displaces any residual air being present in the catheter prior to an advancement of the electrode. If, however, the electrode is to be implanted within muscle tissue or subcutaneously, an air pocket at the tip of the electrode is regularly formed during advancement of the electrode. After removing the delivery catheter, the electrode tip is then positioned within an air cushion, resulting in a decreased sensitivity of the electrode.

According to prior art, air inclusions between an electrode and tissue are tried to be removed by flushing with a saline solution. For this purpose, a sodium chloride solution is injected into an electrode channel or an electrode catheter before the electrode is advanced. Often, it is also tried to displace the air by rather undifferentiated pressing the air with the fingers in the direction of the incision used for implanting the electrode. However, often at least small air cushions cannot be removed by this technique.

Flushing the catheter with a flushing liquid in order to remove residual air is a technique that has been adapted from endovascular implantation. However, in case of implantations into tissue, this technique is less appropriate. This is due to the fact that the sodium chloride solution is not injected into the bloodstream, but rather into the tissue. There, it is drained between the individual tissue layers and does not apply a counter pressure. Thus, it will drain into the tissue and will exit the catheter after removing the syringe used for injecting the solution. Air will be reintroduced into the catheter prior to advancing the electrode to be implanted. As a result, an air cushion around the implanted electrode remains. These air inclusions represent an increased risk for an inadequate or ineffective therapy in many medical applications, such as in case of operating an implantable cardioverter-defibrillator (ICD) like a subcutaneous ICD (S-ICD).

US 2009/0182287 A1 describes devices, systems, and methods for the localization of body lumen junctions and other intraluminal structure. Several of the described embodiments permit clinicians to identify the locations of intraluminal structures and medical devices during non-surgical medical techniques, such as cardiac ablation, by determining the intralumen conductance and/or cross-sectional area at a plurality of locations within the body lumen.

US 5,336,252 A describes a system and method for implanting electric leads in the pericardial space of a heart. The system includes a suction cup mounted to the distal end of a guiding catheter. A vacuum pump evacuates the guiding catheter whereby the suction cup is held against the pericardium. An endoscope fitted through the guiding catheter is used to observe the surface of the pericardium. Then, a channel is formed with the help of a dilator. Finally, an electric lead is advanced through the channel to penetrate the pericardial space and is secured therein with an anchor flange at the end of the electrical lead.

US 2001/0020167 A1 describes systems, apparatus and methods for selectively applying electrical energy to body tissue in order to ablate, contract, coagulate, or otherwise modify a tissue or organ of a patient.

It is an object of the present invention to reduce or even prevent the risk of air inclusions upon the implantation of electrodes in a non-transvenously manner.

This object is achieved with an implantable electrode for a medical device having the features of claim 1. Such an implantable electrode comprises an electrode shaft, an interior lumen inside the electrode shaft and at least one electrode pole on an outer surface of the electrode shaft.

According to an aspect of the present invention, the electrode pole comprises an opening. This opening allows a fluid communication between an outside of the electrode shaft and the interior lumen. The opening can also be denoted as through-hole, aperture or penetration. The interior lumen further comprises a suction port that allows applying a negative pressure to the interior lumen. Thus, the implantable electrode allows the application of negative pressure (vacuum) to its interior lumen and through the opening to an outside of the electrode shaft in the region of the electrode pole. Due to this arrangement, it is possible to draw any residual air being present around the electrode pole into the interior lumen of the electrode shaft and thus away from the electrode pole. As a result, the electrode pole will no longer be surrounded by an air cushion. Rather, it can get into close contact with the surrounding tissue. Thus, it will be able to sense signals from the tissue and stimulate the tissue in a very efficient way with low detection and stimulation thresholds.

Therefore, the implantable electrode allows a much more sensitive detection of electrical signals and a much more sensitive stimulation of surrounding tissue than prior art electrodes do. Sensing artefacts and inadequate stimulation shocks are efficiently reduced or prevented. Reduction or prevention of undesired air inclusions facilitates the implantation of the electrode and the follow-up care of the patient (since the electrode is able to properly sense electric signals right from the start) and ameliorates the remedy process after implantation.

In an embodiment, the implantable electrode comprises a plurality of electrode poles on the outer surface of the electrode shaft. The term "plurality" as used herein refers to a multiplicity of items, such as 2, 3, 4, 5, 6, 7, 8, 9, 10, or more than 10 items. Each electrode pole comprises an opening allowing a fluid communication between an outside of the electrode shaft and the interior lumen. Thus, this embodiment allows the prevention of air inclusions around all electrode poles provided on the electrode shaft. This embodiment enhances the sensitivity of the electrode in a particular appropriate way since it optimizes the contact between each of the electrode poles of the electrode and the surrounding tissue in a particular appropriate manner.

In an embodiment, the implantable electrode comprises a plurality of openings in at least one electrode pole. In this embodiment, the implantable electrode further comprises a plurality of interior lumina. Each opening allows a fluid communication between an outside of the electrode shaft and one of the plurality of interior lumina. Thus, an individual suction lumen is present for each of the plurality of openings, wherein the openings may be provided on a single electrode pole or on a plurality of electric poles.

In an embodiment, the plurality of interior lumina merge into a single interior lumen at a merging site being located proximally to the most proximal opening. Due to the merging of the individual interior lumina to a single interior lumen, it is possible to apply a negative pressure to only this merged single interior lumen, but being nonetheless able to transfer the negative pressure to each of the plurality of interior lumina.

In an embodiment, the electrode pole - or if more than one electrode pole is provided on the electrode shaft - at least one electrode pole of the plurality of electrode poles on the outer surface of the electrode shaft comprises a plurality of openings. In this embodiment, each opening allows a fluid communication between an outside of the electrode shaft and the interior lumen. Thus, a plurality of openings may not only be connected to individual interior lumina, but can also lead into one and the same interior lumen. Such a design can be realized in a particularly easy manner and thus facilitates the manufacturing of the electrode.

In an embodiment, the opening has a diameter lying in a range of from 0.05 mm to 1 mm, in particular of from 0.1 mm to 0.9 mm, in particular of from 0.2 mm to 0.8 mm, in particular of from 0.3 mm to 0.7 mm, in particular from 0.4 mm to 0.6 mm, in particular of from 0.45 mm to 0.5 mm. Generally, a plurality of small openings (having a diameter lying in a lower range of the before-mentioned ranges, e.g., up to a diameter of 0.3 mm) provided over an area of the electrode pole are particularly appropriate for drawing air from an outside of the electrode shaft into the interior lumen upon a negative pressure is applied to the interior lumen.

In an embodiment, the interior lumen has an inner diameter lying in a range of from 0.2 mm to 1 mm, in particular of from 0.3 mm to 0.9 mm, in particular of from 0.4 mm to 0.8 mm, in particular of from 0.5 mm to 0.7 mm, in particular of from 0.55 mm to 0.6 mm. If only a single interior lumen is provided, the diameter of this interior lumen can be larger than in case of providing a plurality of individual interior lumina to be connected to individual openings in the electrode shaft in the region of the electrode pole.

In an embodiment, the opening is arranged in the electrode pole in a radial manner, i.e., facing radially outwards from the interior lumen.

In an embodiment, the implantable electrode comprises a mesh or screen covering the opening. This mesh or screen serves for preventing particulate material from entering the interior lumen through the opening. As explained above, the opening is intended for drawing gas such as air into the interior lumen. However, no particulate material is intended to be aspired by the applied negative pressure. Rather, such particulate material might get stuck in the interior lumen and impair the functioning of the electrode. The mesh can be applied to an outside of the opening (i.e., on an outer surface of the electrode pole). Alternatively, it may be applied to an inside of the interior lumen so that it covers a side of the opening that is oriented towards the interior lumen. Such an arrangement is particularly appropriate since it avoids any additional material on the electrode pole that might influence the electric properties of the electrode pole and therefore of the whole electrode. If a plurality of openings is provided within the electrode shaft, a single piece of mesh can be arranged inside the interior lumen so as to cover all or at least a part of these openings at the same time.

In an embodiment, the implantable electrode comprises a means for preventing gas from exiting the interior lumen through the opening. A particular appropriate embodiment for such a means for preventing gas from exiting the interior lumen through the opening is a valve such as a check valve. Gas may pass the valve in one direction (namely from an exterior of the electrode towards the interior lumen), but not in the opposite direction. Then, it is guaranteed that no gas like air is reintroduced into a surrounding of the electrode upon releasing the negative pressure.

In an embodiment, the implantable electrode comprises a membrane covering the opening. In this context, the membrane is permeable for gases, but impermeable for liquids. Such membranes can be made from expounded polytetrafluoroethylene (ePTFE) like the membranes that are marketed under the trademarks Gore-Tex and Sympatex. Such membrane does not only prevent particulate material from entering the interior lumen, but also liquids. Therefore, such a membrane serves in a particular appropriate way for keeping clean the interior lumen of the electrode and for aspirating only gases like air from the surrounding area of the electrode through the interior lumen.

In an embodiment, the implantable electrode comprises a flushing lumen (being separated from the interior lumen) inside the electrode shaft. Furthermore, it comprises a flushing opening allowing a fluid communication between the flushing lumen and an outside of the electrode shaft. Due to this arrangement, the flushing lumen and the flushing opening allow a provision of a flushing liquid to an outside of the electrode shaft. In this embodiment, the flushing liquid like a sodium chloride solution can be provided through the flushing lumen and the flushing opening towards the site of implantation of the electrode. It can then be fully or partially aspirated through the opening and the interior lumen of the electrode shaft (provided that the opening is not covered by a membrane preventing a liquid from entering the interior lumen). By providing such a flushing liquid, it is possible to reduce the frictional forces upon advancing the electrode and to keep the openings clean due to a permanent flow of liquid or fluid through the openings from an outside of the electrode shaft to the interior lumen, when implanting the implantable electrode.

In an embodiment, the implantable electrode is an epicardial electrode. In an embodiment, the implantable electrode is an electrode of an electrophysiology system. In an embodiment, the electrode is an antenna of a biomonitoring device.

In an aspect, the present invention relates to an electrode assembly that comprises an implantable electrode according to the preceding explanations and a suction device operatively coupled to the suction port of the implantable electrode. This suction device serves for applying a negative pressure to the interior lumen. Thus, the electrode assembly is particularly helpful upon implanting the implantable electrode. Appropriate suction devices are a syringe, a pump, bellows, a suction device of an anesthesia system, or a vacuum reservoir.

In an embodiment, the suction device is coupled to the implantable electrode at a proximal end of the implantable electrode (e.g., in a region in which a connector of the electrode is arranged).

In an embodiment, the suction port of the interior lumen is arranged directly at the proximal end of the electrode shaft. In an embodiment, the suction port is oriented along a longitudinal direction of extension of the electrode shaft, i.e., facing axially outwards from the interior lumen.

In an embodiment, a connection between the implantable electrode and the suction device is established with the help of an adapter. Such an adapter can facilitate the coupling between the suction device and the implantable electrode and serves for a higher number of already existing suction devices that are compatible with the implantable electrode.

In an aspect, the present invention relates to a medical device that comprises an implantable electrode according to the preceding explanations or an electrode assembly according to the preceding explanations.

In an embodiment, the medical device is an active implantable medical device. The term "active implantable medical device" is well known to a person skilled in the art. An "active medical device" is typically defined to be any medical device relying for its functioning on a source of electrical energy or any source of power other than that directly generated by the human body or gravity.

An "active implantable medical device" is typically defined to be any active medical device which is intended to be totally or partially introduced, surgically or medically, into the human body or by medical intervention into a natural orifice, and which is intended to remain after the procedure.

Further details on active implantable medical devices can be found, e.g., in the consolidated text of the Council Directive 90/385/EEC of 20 June 1990 on the approximation of the laws of the Member States relating to active implantable medical devices with subsequent amendments in the version published on 11 October 2007. The consolidated text of this Council Directive is freely accessible under the following link: https://eur-lex.europa.eu/legal-content/EN/TXT/?uri=CELEX:01990L03 85-20071011

In an embodiment, the active implantable medical device is an implantable pulse generator (IPG), an implantable cardioverter-defibrillator (ICD), in particular a subcutaneous ICD (S-ICD), a device for cardiac resynchronization therapy (CRT), or a neurostimulation device.

In an aspect, the present invention relates to a method for implanting an implantable electrode according to the preceding explanations to a patient in need thereof. This method comprises the steps explained in the following.

In one of the method steps, a delivery catheter comprising a delivery channel and a guiding tool inserted into the delivery channel is pushed (or advanced) to a site of implantation. The guiding tool can be, e.g., a guidewire or a trocar.

Afterwards, the guiding tool is removed from the delivery channel, making the delivery channel ready for receiving the implantable electrode.

In a further method step, a negative pressure is applied to an interior lumen of the implantable electrode.

In a further method step, the implantable electrode is inserted into the delivery channel.

Afterwards, the implantable electrode is advanced within the delivery channel towards the site of implantation. The negative pressure applied to the interior lumen of the implantable electrode is transferred through the opening in at least one electrode pole of the electrode to an outside of the implantable electrode. Therefore, the electrode is not only moved by pushing it from a proximal end of the electrode, but also by a suction force acting upon a more distally located region of the electrode. Due to this synergistic effect of forces acting upon the electrode upon implantation, also very soft electrodes can be advanced to the intended site of implantation in a particularly gentle manner.

Afterwards, the delivery catheter is removed and the negative pressure applied to the interior lumen is released.

These method steps need not necessarily be performed in the indicated sequence. Rather, any other sequence may also be adapted, wherein some of the explained steps require that another method step is performed beforehand, as will be immediately apparent for a person skilled in the art.

All embodiments of the implantable electrode can be combined in any desired manner and can be transferred either individually or in any arbitrary combination to the electrode assembly, to the medical device and to the described method. Likewise, all embodiments of the electrode assembly can be combined in any desired manner and can be transferred either individually or in any arbitrary combination to the implantable electrode, to the medical device and to the method. Furthermore, all embodiments of the medical device can be combined in any desired manner and can be transferred either individually or in any arbitrary combination to the implantable electrode, to the electrode assembly and to the described method. Finally, all embodiments of the described method can be combined in any desired manner and can be transferred either individually or in any arbitrary combination to the described implantable electrode, to the electrode assembly and to the medical device.

Further details of aspects of the present invention will be explained referring to exemplary embodiments and accompanying Figures. In the Figures:
- Fig. 1: shows a first embodiment of an implantable electrode and
- Fig. 2: shows a second embodiment of an implantable electrode.

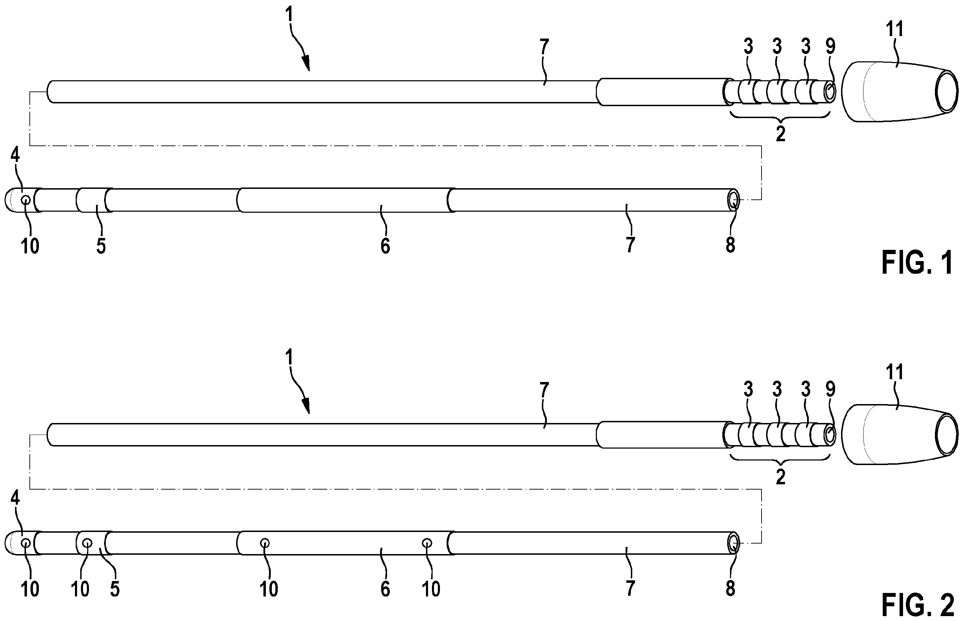

Fig. 1 shows a first embodiment of an implantable electrode 1, wherein the upper portion of Fig. 1 depicts the proximal region of the electrode 1, and the lower part of Fig. 1 depicts the distal region of the electrode 1.

In a proximal end region of the electrode 1, a connecting plug 2 comprising three connecting poles 3 is located. One of the connecting poles 3 is electrically connected to a tip electrode 4. Another of the connecting poles 3 is electrically connected to a ring electrode 5. Yet another one of the connecting poles 3 is electrically connected to a shock electrode 6. The tip electrode 4, the ring electrode 5 and the shock electrode 6 constitute electrode poles of the electrode 1. They are located on an outer surface of an electrode shaft 7 of the electrode 1.

In its interior, the electrode shaft 7 comprises an inner lumen 8 which is accessible through a proximal suction port 9 located at the very proximal end of the electrode 1, i.e., at the proximal terminus of the connector plug 2. The tip electrode 4 comprises an opening 10 that is in fluid communication with the inner lumen 8.

An adapter 11 is placed over the connector plug 2 and allows a connection of a suction device to the electrode 1. Negative pressure applied by such a suction device will then be transferred to the inner lumen 8 and finally to an outside of the opening 10. When implanting the electrode 1 and applying a negative pressure to the inner lumen 8, any residual air being around the tip electrode 4 will be aspirated due to the negative pressure through the opening 10 to the inner lumen 8 and finally to the suction device. Therefore, no air cushion will be present around the tip electrode 4 after having completed the implantation of the electrode 1. As a result, the electrode 1 - and particularly its tip electrode 4 - can be operated with a high sensitivity due to the absence of any undesired air inclusions. Rather, a direct contact between the tip electrode 4 and the surrounding tissue is made possible.

The adapter 11 serves for a fluid communication between the suction device and the inner lumen 8, wherein this communication is sealed to an outside of the electrode 1.

As will be apparent from the preceding explanations, it might still be possible that residual air is accumulated around the ring electrode 5 or the shock electrode 6. While air inclusions around these electrodes are not that often like air inclusions around the tip electrode 4, embodiments of the present invention also address potential problems with air inclusions at other electrodes than the tip electrode 4. This will be explained with respect to Fig. 2.

In Fig. 2, another embodiment of an electrode 1 is shown. Similar elements will be denoted with the same numeral references as in Fig. 1.

The electrode 1 shown in Fig. 2 is almost identical to the electrode 1 shown in Fig. 1. It also comprises an electrode shaft 7, an inner lumen 8, a tip electrode 4, a ring electrode 5, and a shock electrode 6. However, in contrast to the embodiment shown in Fig. 1, the electrode 1 of Fig. 2 does not only comprise an opening 10 in the tip electrode 4, but also an opening 10 in the ring electrode 5 as well as two openings 10 in the shock electrode 6. Each of these openings 10 serves for a fluid communication between an outside of the electrode 1 and the inner lumen 8.

If a negative pressure is applied by a suction device to the inner lumen 8, this negative pressure will be transferred to an outside of each of the openings 10. Consequently, any residual air being present around the tip electrode 4, the ring electrode 5 and/or the shock electrode 6 will be drawn through the opening 10 into the inner lumen 8 and finally to the suction device.

Thus, this embodiment comprises different electrode poles 4, 5, 6, wherein at least one opening 10 is present in each of these electrode poles 4, 5, 6. Consequently, any air inclusions around the tip electrode 4, the ring electrode 5, and/or the shock electrode 6 will be efficiently removed during implantation of the electrode 1. This will serve for a particular reliable and sensitive operation of the electrode 1 after having been implanted.

## Claims

1. Implantable electrode for a medical device, the implantable electrode (1) comprising an electrode shaft (7), an interior lumen (8) inside the electrode shaft (7) and an electrode pole (4, 5, 6) on an outer surface of the electrode shaft (7),
**characterized**
**in that** the electrode pole (4, 5, 6) comprises an opening (10) allowing a fluid communication between an outside of the electrode shaft (7) and the interior lumen (8), wherein the interior lumen (8) comprises a suction port (9) allowing to apply a negative pressure to the interior lumen (8).

2. Implantable electrode according to claim 1, **characterized in that** the implantable electrode (1) comprises a plurality of electrode poles (4, 5, 6) on the outer surface of the electrode shaft (7), wherein each electrode pole (4, 5, 6) comprises an opening (10) allowing a fluid communication between an outside of the electrode shaft (7) and the interior lumen (8).

3. Implantable electrode according to claim 1, **characterized in that** the implantable electrode (1) comprises a plurality of openings (10) in at least one electrode pole (4, 5, 6) and a plurality of interior lumina, wherein each opening (10) allows a fluid communication between an outside of the electrode shaft (7) and one of the plurality of interior lumina.

4. Implantable electrode according to claim 3, **characterized in that** the plurality of interior lumina merge to a single interior lumen (8) proximal to the most proximal opening (10).

5. Implantable electrode according to claims 1 or 2, **characterized in that** the electrode pole (4, 5, 6) or at least one electrode pole (4, 5, 6) of a plurality of electrode poles (4, 5, 6) on the outer surface of the electrode shaft (7) comprises a plurality of openings (10), each opening (10) allowing a fluid communication between an outside of the electrode shaft (7) and the interior lumen (8).

6. Implantable electrode according to any of the preceding claims, **characterized in that** the opening (10) has a diameter lying in a range of from 0.05 mm to 1 mm.

7. Implantable electrode according to any of the preceding claims, **characterized in that** the interior lumen (8) has an inner diameter lying in a range of from 0.2 mm to 1 mm.

8. Implantable electrode according to any of the preceding claims, **characterized in that** the implantable electrode (1) comprises a mesh covering the opening (10) and serving for preventing particulate material from entering the interior lumen (8) through the opening (10).

9. Implantable electrode according to any of the preceding claims, **characterized in that** the implantable electrode (1) comprises a means for preventing gas from exiting the interior lumen (8) through the opening (10).

10. Implantable electrode according to any of the preceding claims, **characterized in that** the implantable electrode (1) comprises a membrane covering the opening (10) and being permeable for gases, but impermeable for liquids.

11. Implantable electrode according to any of the preceding claims, **characterized in that** the implantable electrode (1) comprises a flushing lumen inside the electrode shaft (7) and a flushing opening allowing a fluid communication between the flushing lumen and an outside of the electrode shaft (7), the flushing lumen and the flushing opening allowing a provision of a flushing liquid to an outside of the electrode shaft (7).

12. Electrode assembly, comprising an implantable electrode (1) according to any of the preceding claims and a suction device operatively coupled to the suction port (9) of the implantable electrode (1).

13. Medical device, comprising an implantable electrode (1) according to any of claims 1 to 11 or an electrode assembly according to claim 12.

14. Medical device according to claim 13, **characterized in that** the medical device is an active implantable medical device.

15. Method for an implanting an implantable electrode according to any of claims 1 to 11 to a patient in need thereof, the method comprising the following steps:
a) pushing a delivery catheter comprising a delivery channel and a guiding tool inserted into the delivery channel to a site of implantation;
b) removing the guiding tool from the delivery channel;
c) applying a negative pressure to an interior lumen (8) of the implantable electrode (1);
d) inserting the implantable electrode (1) into the delivery channel;
e) advancing the implantable electrode (1) within the delivery channel towards the site of implantation;
f) removing the delivery catheter; and
g) releasing the negative pressure.
